# EUROPEAN PATENT APPLICATION

(11) **EP 3 764 103 A1**
(43) Date of publication of application: **13.01.2021**
(21) Application number: 19315069.5
(22) Date of filing: 12.07.2019
(51) Int. Cl.: G01N 33/92

(54) **METHOD OF DIAGNOSIS OR PROGNOSIS OF A NON-HEALING OR CHRONIC WOUND**

(71) Applicant: URGO RECHERCHE INNOVATION ET DEVELOPPEMENT, 21300 Chenôve (FR)
(72) Inventor: HOLMES Elaine, LONDON SW7 2AZ (GB); SPAGOU Konstantina, THAME OX9 3DF (GB); VELINENI Rahul, GLASGOW G41 3DA (GB); ONIDA Sarah, LONDON W6 8RF (GB); DAVIES Alun Huw, LONDON W6 8RF (GB); BERGNER Richmond Takyi, BREMEN 28327 (DE); MANJIT Gohel, CAMBRIDGE CB 22 5UT (GB)
(74) Representative: Plasseraud IP

(57) **Abstract**

The present invention relates to a method of diagnosis or prognosis of a non-healing or chronic wound comprising the step of determining the ratio of carnitine to ceramide and/or ceramide derivative in a sample, or the level of at least one metabolite in a sample.

## Description

### FIELD OF THE INVENTION

The present invention relates to a method of diagnosis or prognosis of wounds of mammalian, in particular in human. The said wound is preferably a venous leg ulcer. The method determines a metabolite signature in order to distinguish between a non-pathological wound healing and a pathological wound healing (such as chronic or non-healing wound).

### BACKGROUND OF THE INVENTION

Chronic or non-healing wounds are a worldwide health problem, in part due to a lack of adequate methods of treatment. In 2010, more than 7 million people worldwide suffered from chronic wounds, and the projected annual increase is at least 10 percent.

Non-healing wounds or chronic wounds are a challenge for the patient, the health care professional, and the health care system. They significantly impair the quality of life for millions of people and impart burden on society in terms of lost productivity and health care money.

Wound healing is a dynamic pathway that leads to the restoration of tissue integrity and functions. A chronic wound or non-healing wound develops when the normal reparative process is disturbed. By understanding the biology of wound healing, the physician can optimize the wound healing by choosing the adequate treatment. Venous leg ulcers are an example of a common type of chronic or non-healing wounds.

Venous leg ulceration (VLU) or chronic venous ulceration affects 1% of the population, increasing to 4% in those over the age of 65 (Callam et al. 1985). Despite its relatively low prevalence, VLU is responsible for up to 2% of the annual healthcare budget expenditure in western societies, presenting an important economic burden (Gottrup et al. 2001). It is also a significant burden to patients, with quality of life effects as important as those experienced by individuals with severe chronic conditions, such as congestive cardiac failure or chronic obstructive pulmonary disease (Broszczak et al 2017). So far, compression has been the mainstay of ulcer treatments, notably VLU, but it can be painful and time-consuming. Besides, ulcers are difficult to heal and compression is not always delivered effectively in the community (Hampton, 2016). Furthermore, up to 20% of ulcers recur within 1 year despite adequate treatment; the mechanisms for this are unclear (Gohel et al. 2005; Broszczak et al. 2017).

Also, the recent Early Endovenous Ablation in Venous Ulceration trial (EVRA) reported that intervening on incompetent veins early can reduce time to ulcer healing and increase ulcer free time (Gohel et al. 2018). Despite this, there is still urgent need for supportive measures to manage this condition, particularly in light of its prevalence in the elderly population, which is predicted to expand significantly in the coming years with a corresponding increase in venous disease (Onida et al. 2016).

Recent research has focused on understanding the molecular biology of the formation of VLUs and their ability to re-epithelialise and ultimately heal. Omics technologies such as genomics, transcriptomics and proteomics have highlighted important cellular pathways of relevance in VLU, although these have failed to translate into clinically significant interventions (Broszczak et al. 2017). Metabonomics, the study of the end products of cellular metabolism, enables qualitative and quantitative assessment of molecules associated with a disease process, helping develop novel biomarkers. This information is complementary to what has been discovered via other omic technologies and can help develop diagnostic and therapeutic applications to improve the quality of care delivered to patients. However, compared to the other omic platforms, only a few metabonomic studies have investigated venous leg ulceration. The important metabolites highlighted in vein leg ulcer research include L-arginine, nitric oxide, iron and free radicals, which are increased in ulcer fluid from patients with chronic wounds (Ennis et al. 1994, Trengove et al. 1996, Zemstov et al. 1995, James et al. 2003, Kalkhof et al. 2014, Junka et al. 2017). These results agree with proteomic studies confirming an inflammatory phenotype in chronic wounds (Mannello et al. 2014). However, only a limited number of molecules have currently been associated with chronic wounds, let alone with VLU, and none of them are established for use in a standard clinical care setting, notably as a reliable method of diagnosis or prognosis of the outcome of a wound.

Therefore, there is a need to provide a reliable method of diagnosis or prognosis of the outcome of a wound, notably an ulcer. There is notably a need to identify biomarkers predictive of healing or failure to heal in a venous leg ulcer patient. This identification of biomarkers, as well as the method relied upon, is needed for disease prognostication and the development of novel therapy, in order to adapt the best treatment to provide the wound healing. There is also a need for a better care of the wound and for the reduction of the deadline of said care.

### SUMMARY OF THE INVENTION

The present invention relates to the results from the inventors who performed untargeted metabolic profiling on urine, serum, and ulcer fluid samples, using liquid chromatography-mass spectrometry and nuclear magnetic resonance spectroscopy. Following multivariate and univariate analysis, they identified differential metabolic phenotypes in healing compared with nonhealing VLU patients. Thus, statistically significant metabolites responsible for defining the metabolic phenotype were identified.

The present invention relates then to the results from the inventors showing that the ceramide, ceramide derivatives, carnitine and phosphatidylethanolamine can be used as biomarkers predictive of healing or failure to heal in a wound, notably a venous leg ulcer. The inventors have notably identified a ratio of carnitine to ceramide and/or ceramide derivative useful for diagnosis or prognosis of a non-healing or chronic wound, notably a venous leg ulcer.

Therefore, the present invention relates to a method of diagnosis or prognosis of a non-healing or chronic wound comprising the step of determining the ratio of carnitine to ceramide and/or ceramide derivative in a sample from a mammalian, wherein said ratio is decreased when compared with a reference value.

The present invention also relates to a method of diagnosis or prognosis of a non-healing or chronic wound comprising the step of determining the level of at least one metabolite in a sample from a mammalian, wherein:
- at least carnitine is lower than a reference value, and/or
- at least one ceramide and/or one ceramide derivative is higher than a reference value, and/or
- at least one phosphatidylethanolamine is higher than a reference value.

According to the invention, the said ceramide is preferably Cer d18:1/24:0, Cer d18:1/24:1, Cer d18:1/23:0, Cer d18:2/23:0, Cer d18:1/22:0, and/or Cer d18:2/22:0.

According to the invention, the said ceramide derivative is preferably ceramide-1-phosphate or sphingomyelin.

According to the invention, the said ceramide-1-phosphate is preferably CerP d18:1/18:0.

According to the invention, the said sphingomyelin is preferably SM d18:1/23:0.

According to the invention, the said phosphatidylethanolamine is preferably PE 18:4/22:6.

According to the invention, the said wound is preferably an ulcer, preferably a leg ulcer, more preferably a venous leg ulcer or a chronic venous ulceration.

According to the invention, the said sample is preferably chosen among: blood, serum, urine and ulcer fluids, preferably serum, urine and ulcer fluids, more preferably serum.

### DETAILED DESCRIPTION OF THE INVENTION

The present invention first relates to a method of diagnosis or prognosis of a non-healing or chronic wound comprising the step of determining the ratio of carnitine to ceramide and/or ceramide derivative in a sample from a mammalian, wherein said ratio is decreased when compared with a reference value.

According to the invention, the term "carnitine" refers to the compound also named β-hydroxy-γ-N-trimethylaminobutyric acid, 3-hydroxy-4-N,N,N-trimethylaminobutyrate, and preferably refers to L-carnitine. Carnitine is an important nutrient that contributes to energy metabolism and fatty acid metabolism.

According to the invention, the term "ceramide" refers to a lipid that is made up of at least one sphingosine attached to a fatty acid. Ceramides are notaby referenced under the Structure Database (LMSD) of the Lipid MAPS® Lipidomics Gateway. According to the invention, the term "ceramide" also encompasses the term "ceramides"

According to the invention, the term "ceramide derivative" refers to a compound that is made up of at least a ceramide unit. The "ceramide unit" can for example be linked to another compound such as phosphocholine, or be phosphorylated. Therefore, in a preferred embodiment of the invention, the said ceramide derivative is a ceramide-1-phosphate or a sphingomyelin. According to the invention, the term "ceramide derivative" also encompasses the term "ceramide derivatives".

According to the invention, the "ratio of carnitine to ceramide and/or ceramide derivative" means the level of carnitine in a sample divided by the level of ceramide and/or ceramide derivative in the same sample. Preferably, this ratio corresponds to the level of carnitine in a sample divided by the level of ceramide and ceramide derivative in the same sample.

In a preferred embodiment of the invention, said method of diagnosis or prognosis of a non-healing or chronic wound comprises the following steps:
a) determining the ratio of carnitine to ceramide and/or ceramide derivative in a sample from a mammalian,
b) comparing the said ratio to a reference value,
c) providing a favorable diagnosis or prognosis of healing when the said ratio is higher than said reference value, or providing a poor diagnosis or prognosis of healing when the said ratio is lower than said reference value.

In a preferred embodiment of the invention, the reference value is 40, and has notably been determined by the method of the threshold value. Therefore, this value has been determined according to a general guide that defines the true threshold or cut-off as the value which divides the population (healers and non-healers) into two subsets, and that this subset is as large as possible. This method has been described in Spencer et al., Stat Med. 2016 November 30; 35(27): 4909-4923. Said reference is preferably predetermined, and typically corresponds to a value which has been determined in people who healed. In a preferred embodiment of the invention, this reference value has been determined in a population of persons suffering from a venous leg ulcer who healed. Also, in a preferred embodiment of the invention, the reference value is determined after a 20 weeks follow-up of the wound (preferably from the appearance of the first clinical signs of the wound).

In a preferred embodiment of the invention, said method of diagnosis or prognosis of a non-healing or chronic wound is an *in vitro* method.

In a preferred embodiment of the invention, said method of diagnosis or prognosis of a non-healing or chronic wound comprises a step of obtaining a sample from a mammalian. Said step is performed before the step a) as defined above. In a preferred embodiment of the invention, said step a) as defined above is a step of determining the ratio of carnitine to ceramide and/or ceramide derivative in a sample which has been obtained from a mammalian. In a preferred embodiment of the invention, said determining step a) is performed after a between one and 20 weeks follow-up of the wound, preferably from the appearance of the first clinical signs of the wound.

In a preferred embodiment of the invention, said method of diagnosis or prognosis of a non-healing or chronic wound further comprises a step of identifying the ceramide and/or ceramide derivative present in the sample, notably in order to perform a metabolic profiling analysis. Such step if preferably performed before the step of determining the ratio of carnitine to ceramide and/or ceramide derivative in a sample from a mammalian. Therefore, in a most preferred embodiment, of the invention, said method of diagnosis or prognosis of a non-healing or chronic wound comprises the following steps:
a) optionally, obtaining a sample from a mammalian,
b) identifying the ceramide and/or ceramide derivative present in said sample,
c) determining the ratio of carnitine to ceramide and/or ceramide derivative in said sample,
d) comparing the said ratio to a reference value, and
e) providing a favorable diagnosis or prognosis of healing when the said ratio is higher than said reference value, or providing a poor diagnosis or prognosis of healing when the said ratio is lower than said reference value.

In a preferred embodiment of the invention, the step of identifying the ceramide and/or ceramide derivative present in said sample and/or determining the ratio can be performed by any known techniques from the skilled person, using one or several techniques such as NMR (such as ¹H-NMR), reversed phase UPLC-MS (RP-UPLC-MS) in electrospray ionization (ESI+ and ESI- modes) and hydrophilic interaction liquid chromatography UPLC-MS (HILIC-UPLC-MS) in electrospray ionization ESI+ and ESI- modes, as well as multivariate data analysis (MVDA) techniques and univariate data analysis, such as principal component analysis (PCA), partial least squares (PLS) and orthogonal PLS (OPLS), O2PLS or the SIMCA method. In a most preferred embodiment of the invention, the step of identifying and/or determining is performed using liquid chromatography-mass spectrometry and optionally nuclear magnetic resonance spectroscopy, as well as multivariate and univariate analysis. In a preferred embodiment of the invention, the step of identifying the ceramide and/or ceramide derivative present in said sample and/or determining the ratio can be performed by using reversed phase UPLC-MS (RP-UPLC-MS) in electrospray ionization (ESI+ and ESI- modes) or hydrophilic interaction liquid chromatography UPLC-MS (HILIC-UPLC-MS) in electrospray ionization ESI+ and ESI- modes and orthogonal PLS (OPLS). In another preferred embodiment of the invention, the step of identifying is performed with NMR, reversed-phase UPLC-MS in ESI+ and ESI- modes and/or HILIC-UPLC-MS in ESI+ and ESI- modes. In a preferred embodiment of the invention, the step of identifying and/or determining is performed with NMR, reversed-phase UPLC-MS in ESI+ and ESI- modes and/or HILIC-UPLC-MS in ESI+ and ESI- modes, preferably combined with multivariate data analysis. In a most preferred embodiment of the invention, the step of identifying and/or determining is performed with reversed-phase UPLC-MS in ESI+ and ESI- modes and/or HILIC-UPLC-MS in ESI+ and ESI- modes, preferably combined with multivariate data analysis, even more preferred from a serum sample. In another most preferred embodiment of the invention, the step of identifying and/or determining ceramide and/or ceramide derivative is performed with reversed-phase UPLC-MS in ESI+ and ESI- modes, preferably combined with multivariate data analysis, and the step of identifying and/or determining carnitine is performed with HILIC-UPLC-MS in ESI+ and ESI- modes, preferably combined with multivariate data analysis, even more preferred from a serum sample.

In a preferred embodiment of the invention, the said ceramide is Cer d18:1/24:0, Cer d18:1/24:1, Cer d18:1/23:0, Cer d18:2/23:0, Cer d18:1/22:0, and/or Cer d18:2/22:0. In a preferred embodiment of the invention, the level at least one of the ceramide chosen among Cer d18:1/24:0, Cer d18:1/24:1, Cer d18:1/23:0, Cer d18:2/23:0, Cer d18:1/22:0, or Cer d18:2/22:0 is determined, preferably 2, 3, 4, 5 or the six, and optionally, the level of other ceramide or ceramide derivative can also determined.

According to the invention, the ceramide "Cer d18:1/24:0" corresponds to N-(tetracosanoyl)-sphing-4-enine ; the ceramide "Cer d18:1/24:1" corresponds to N-(15Z-tetracosenoyl)-sphing-4-enine; the ceramide "Cer d18:1/23:0" corresponds to N-(tricosanoyl)-sphing-4-enine ; the ceramide "Cer d18:2/23:0" corresponds to N-(tricosanoyl)-4E,14Z-sphingadienine; the ceramide "Cer d18:1/22:0" corresponds to N-(docosanoyl)-sphing-4-enine and the ceramide "Cer d18:2/22:0" corresponds to N-(docosanoyl)-4E,14Z-sphingadienine.

In a preferred embodiment of the invention, the said ceramide-1-phosphate is CerP d18:1/18:0.

In a preferred embodiment of the invention, the said sphingomyelin is SM d18:1/23:0.

In a preferred embodiment, in said method of diagnosis or prognosis of a non-healing or chronic wound, the said phosphatidylethanolamine is PE 18:4/22:6

In a preferred embodiment of the invention, the ratio of carnitine to ceramide and/or ceramide derivative corresponds to the following ratio:
- carnitine/(Cer d18:1/24:0, Cer d18:1/24:1, Cer d18:1/23:0, Cer d18:2/23:0, Cer d18:1/22:0, and Cer d18:2/22:0), or
- carnitine/(Cer d18:1/24:0, Cer d18:1/24:1, Cer d18:1/23:0, Cer d18:2/23:0, Cer d18:1/22:0, Cer d18:2/22:0 and CerP d18:1/18:0), or
- carnitine/(Cer d18:1/24:0, Cer d18:1/24:1, Cer d18:1/23:0, Cer d18:2/23:0, Cer d18:1/22:0, Cer d18:2/22:0, CerP d18:1/18:0 and SM d18:1/23:0), or
- carnitine/(CerP d18:1/18:0), or
- carnitine/(SM d18:1/23:0), or
- carnitine/(CerP d18:1/18:0 and SM d18:1/23:0),

In a preferred embodiment of the invention, the said wound is an ulcer, preferably a leg ulcer, more preferably a venous leg ulcer or a chronic venous ulceration..

In a preferred embodiment of the invention, the said sample is chosen among: blood, serum, urine and ulcer fluids, preferably serum, urine and ulcer fluids, and more preferably serum. In a most preferred embodiment of the invention, the sample comprises carnitine, but also Cer d18:1/24:0, Cer d18:1/24:1, Cer d18:1/23:0, Cer d18:2/23:0, Cer d18:1/22:0, Cer d18:2/22:0, CerP d18:1/18:0 and SM d18:1/23:0.

In a second aspect, the present invention also relates to a method of diagnosis or prognosis of a non-healing or chronic wound comprising the step of determining the level of at least one metabolite in a sample from a mammalian, wherein:
- at least carnitine is lower than a reference value, and/or
- at least one ceramide and/or one ceramide derivative is higher than a reference value, and/or
- at least one phosphatidylethanolamine is higher than a reference value.

The definitions mentioned in reference to the first aspect of the invention also applies to the second one.

In a preferred embodiment of this second aspect, the invention relates then to a method of diagnosis or prognosis of a non-healing or chronic wound wherein:
- the said ceramide is Cer d18:1/24:0, d18:1/24:1, d18:1/23:0, d18:2/23:0, d18:1/22:0, and/or d18:2/22:0, and/or
- the said ceramide derivative is:
   * a ceramide-1-phosphate, preferably CerP d18:1/18:0, and/or
   * a sphingomyelin, preferably SM d18:1/23:0, and/or
- the said phosphatidylethanolamine is PE 18:4/22:6.

In a preferred embodiment of this second aspect, the invention relates to a method of diagnosis or prognosis of a non-healing or chronic wound comprising the following steps:
a) determining the level of at least one metabolite, wherein said metabolites is chosen among:
   - carnitine, and/or
   - one ceramide, notably Cer d18:1/24:0, d18:1/24:1, d18:1/23:0, d18:2/23:0, d18:1/22:0, and/or d18:2/22:0, and/or
   - one phosphatidylethanolamine, preferably PE 18:4/22:6, and/or
   - one ceramide derivative, preferaby chosen among is:
      * a ceramide-1-phosphate, preferably CerP d18:1/18:0, and/or
      * a sphingomyelin, preferably SM d18:1/23:0,
b) comparing the said level to a reference value,
c)
   - providing a favorable diagnosis or prognosis of healing when the carnitine level is higher than said reference value, or providing a poor diagnosis or prognosis of healing when the said carnitine level is lower than said reference value, and/or
   - providing a favorable diagnosis or prognosis of healing when the ceramide level and/or ceramide derivative level is lower than said reference value, or providing a poor diagnosis or prognosis of healing when the said ceramide level and/or ceramide derivative level is higher than said reference value, and/or
   - providing a favorable diagnosis or prognosis of healing when the phosphatidylethanolamine level is lower than said reference value, or providing a poor diagnosis or prognosis of healing when the said phosphatidylethanolamine level is higher than said reference value.

In a preferred embodiment of this second aspect, said method also comprises a step of identifying the ceramide, ceramide derivative and/or phosphatidylethanolamine present in the sample, notably in order to perform a metabolic profiling analysis. Such step if preferably performed before the step of determining the level of carnitine, ceramide, ceramide derivative and/or phosphatidylethanolamine in a sample from a mammalian. In another embodiment, the steps of identifying and determining the level of carnitine, ceramide, ceramide derivative and/or phosphatidylethanolamine are simuteaneously performed. Therefore, in a most preferred embodiment, of the invention, said method of diagnosis or prognosis of a non-healing or chronic wound comprises the following steps:
a) optionally, obtaining a sample from a mammalian,
b) identifying the ceramide, ceramide derivative and/or phosphatidylethanolamine present in said sample,
c) determining the level of at least one metabolite, wherein said metabolites is preferably chosen among:
   - carnitine, and/or
   - one ceramide, notably Cer d18:1/24:0, Cer d18:1/24:1, Cer d18:1/23:0, Cer d18:2/23:0, Cer d18:1/22:0, and/or Cer d18:2/22:0, and/or
   - one phosphatidylethanolamine, preferably PE 18:4/22:6, and/or
   - one ceramide derivative, preferaby chosen among is:
      * a ceramide-1-phosphate, preferably CerP d18:1/18:0, and/or
      * a sphingomyelin, preferably SM d18:1/23:0,
d) comparing the said level to a reference value,
e) providing a favorable diagnosis or prognosis of healing when the carnitine level is higher than said reference value, or providing a poor diagnosis or prognosis of healing when the said carnitine level is lower than said reference value, and/or
   - providing a favorable diagnosis or prognosis of healing when the ceramide level and/or ceramide derivative level is lower than said reference value, or providing a poor diagnosis or prognosis of healing when the said ceramide level and/or ceramide derivative level is higher than said reference value, and/or
   - providing a favorable diagnosis or prognosis of healing when the phosphatidylethanolamine level is lower than said reference value, or providing a poor diagnosis or prognosis of healing when the said phosphatidylethanolamine level is higher than said reference value.

In a preferred embodiment of the second aspect, the reference value corresponds to a fold change between the people who healed *vs* non-healing people. In another preferred embodiment, the discriminatory fold changes is as follows: 1.840 for carnitine, 0.790 for Cer d18:1/24:0, 0.738 for Cer d18:1/24:1, 0.722 for Cer d18:1/23:0, 0.781 for Cer d18:2/23:0, 0.744 for Cer d18:1/22:0, 0.656 for Cer d18:2/22:0, 0.782 for CerP d18:1/18:0, 0.654 for SM d18:1/23:0 and/or 0.349 for PE 18:4/22:6, notably as determined with UPLC-MS, preferably in serum.

In a preferred embodiment of the second aspect, the step of identifying the ceramide, ceramide derivative and/or phosphatidylethanolamine present in said sample and/or determining the level of at least one metabolite such as carnitine, ceramide, ceramide derivative and/or phosphatidylethanolamine can be performed by any known techniques from the skilled person, such as NMR (such as ¹H-NMR), reversed phase UPLC-MS (RP-UPLC-MS) in electrospray ionization (ESI+ and ESI- modes) and hydrophilic interaction liquid chromatography UPLC-MS (HILIC-UPLC-MS) in electrospray ionization ESI+ and ESI-modes, as well as multivariate data analysis (MVDA) techniques and univariate analysis, such as principal component analysis (PCA), partial least squares (PLS) and orthogonal PLS (OPLS), 02PLS or the SIMCA method. In a most preferred embodiment of the invention, the step of identifying and/or determining is performed using liquid chromatography-mass spectrometry and optionally nuclear magnetic resonance spectroscopy, as well as multivariate and univariate analysis. In a preferred embodiment of the invention, the step of identifying the ceramide, ceramide derivative and/or phosphatidylethanolamine present in said sample and/or determining the level of at least one metabolite such as carnitine, ceramide, ceramide derivative and/or phosphatidylethanolamine can be performed by using reversed phase UPLC-MS (RP-UPLC-MS) in electrospray ionization (ESI+ and ESI- modes) or hydrophilic interaction liquid chromatography UPLC-MS (HILIC-UPLC-MS) in electrospray ionization ESI+ and ESI- modes and orthogonal PLS (OPLS). In another preferred embodiment of the second aspect, the step of identifying is performed with NMR, reversed-phase UPLC-MS in ESI+ and ESI- modes and/or HILIC-UPLC-MS in ESI+ and ESI- modes. In a preferred embodiment of the invention, the step of identifying and/or determining is performed with NMR, reversed-phase UPLC-MS in ESI+ and ESI- modes and/or HILIC-UPLC-MS in ESI+ and ESI- modes, preferably combined with multivariate data analysis. In a most preferred embodiment of the invention, the step of identifying and/or determining is performed with reversed-phase UPLC-MS in ESI+ and ESI- modes and/or HILIC-UPLC-MS in ESI+ and ESI- modes, preferably combined with multivariate data analysis, even more preferred from a serum sample. In another most preferred embodiment of the invention, the step of identifying and/or determining ceramide and/or ceramide derivative is performed with reversed-phase UPLC-MS in ESI+ and ESI- modes, preferably combined with multivariate data analysis, and the step of identifying and/or determining carnitine and/or phosphatidylethanolamine is performed with HILIC-UPLC-MS in ESI+ and ESI- modes, preferably combined with multivariate data analysis, even more preferred from a serum sample.

The invention will be illustrated by means of the following examples as well as the tables and figures, without being limitative.

### FIGURE LEGENDS

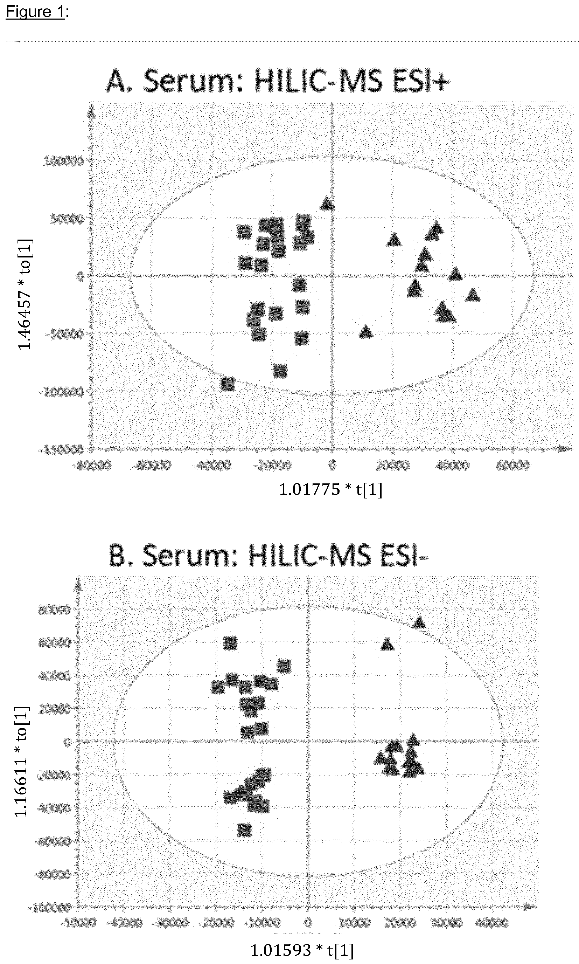

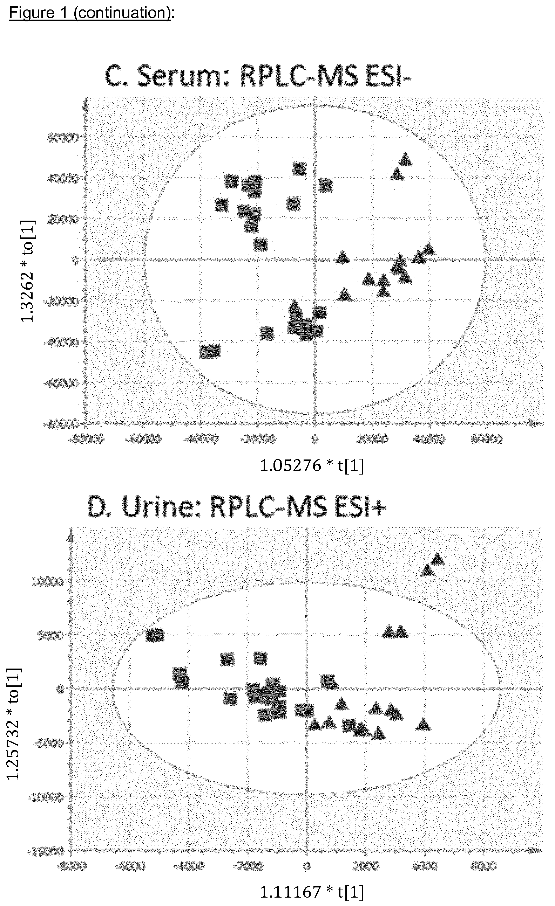

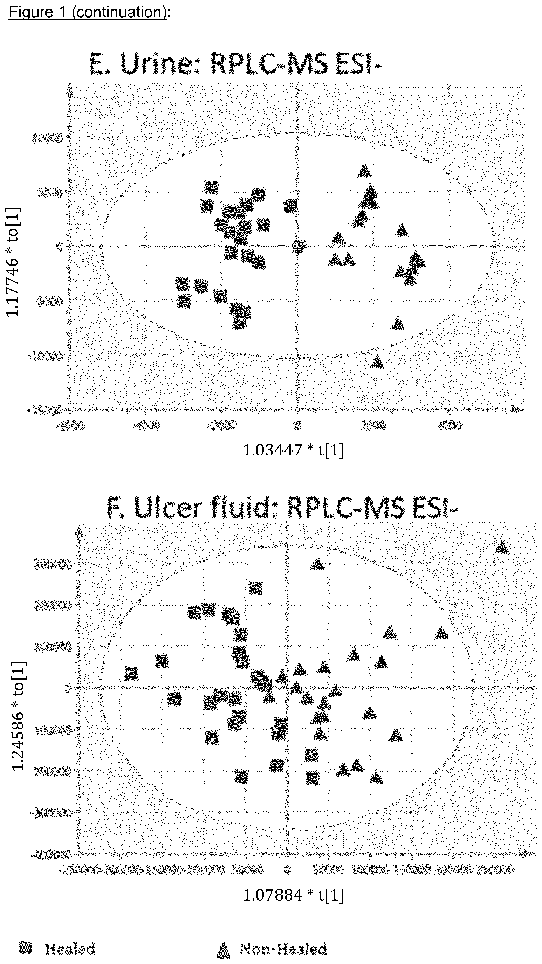

**Figure 1** represents multivariate OPLS-DA cross-validated scores for metabolic profiling of serum, urine and ulcer fluid samples of non-healed(red) and Healed(blue) vein leg ulcer patients. (A) positive mode (ESI+) HILIC-MS and (B) negative mode (ESI-) HILIC-MS profiling analysis of serum; (C) negative mode (ESI-) of lipid RPLC-MS profiling analysis of serum; (D) positive mode (ESI+) RPLC-MS and (E) negative mode (ESI-) of RPLC-MS profiling analysis of urine; (F) negative mode (ESI-) of lipid RPLC-MS profiling analysis of ulcer fluid. The R² and Q² values for OPLS-DA scores plots are shown in **Table 2.**

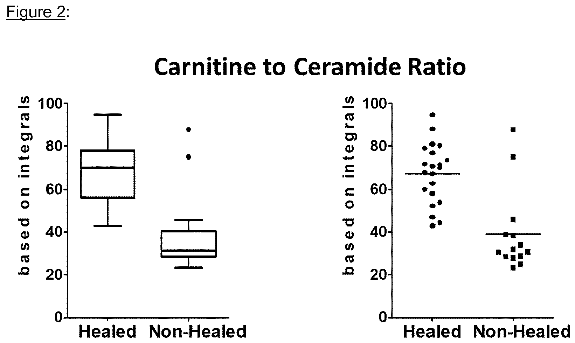

**Figure 2** represents carnitine to ceramide ratio as a predictive biomarker of ulcer healing. Significance was measured via Student's unpaired T-test (p-value<0.0001)

### EXAMPLES

### A - METHODS

### 1. Obtention of the sample

Samples consisting of serum, urine and ulcer fluids were obtained from 28 patients suffering from a venous leg ulcer. After 20 weeks follow up, 15 patients had healed venous leg ulcers and 13 were unhealed.

### 2. Experimental analysis

### Sample preparation and UPLC-MS analysis

Serum and urine samples were analysed by Reversed phase (RP) and hydrophilic interaction liquid chromatography (HILIC) UPLC-MS profiling methods. For ulcer fluid profiling, organic and aqueous metabolites were extracted, for example as described by Anwar et al. 2015. Lipid profiling of serum and ulcer fluid organic metabolite extracts was performed using RP-UPLC-MS as described by Isaac et al. 2011. HILIC-UPLC-MS metabolic profiling of serum and urine was conducted, for example as described by Spagou et al. 2011. The separated samples were analysed with electrospray ionisation (ESI) + and ESI- modes.

### Sample preparation and NMR analysis

For NMR analysis of serum and the ulcer fluid, aqueous extract buffer solution of 0.075 M Na₂HPO₄x7H₂O, 4% sodium azide (NaN₃) in H₂O, 20% deuterium oxide (D₂O), and 0.08% 3-(trimethyl-silyl) propionic acid-d4 (TSP) was added to serum and ulcer fluid aqueous extract samples. For NMR urine analysis, a 0.075M Na₂HPO₄ x 7H₂O, 17 % deuterium oxide (D₂O) containing 0.1% TSP was added to the urine samples. ¹H-NMR data acquisition was performed as previously described by Dona et al. 2014.

### Data processing and statistical analysis

Selected characteristics of the study populations were described and compared according to healing status. Differences between the healing groups were tested using the Kruskal-Wallis one-way analysis of variance.

The raw MS data were collected in centroid mode and converted to netCDF format using the DataBridge tool implemented in MassLynxTM software (Waters Corporation, Milford, USA). The data were pre-processed using the freely available package in R programming software, XCMS (Smith et al. 2006, Tautenhahn et al. 2008) and an output table was obtained comprising pairs of mass/charge ratio (m/z), retention time (RT) and intensity values of the detected metabolite features in each sample. The resulting data underwent total area normalization. Then, univariate statistical analysis was applied to each discriminatory metabolite followed by a two tailed Student t-test with a Benjamini-Hochberg multiple tests (adjusted p-value). An adjusted p-value of less than 0.05 was assigned as significant.

### Metabolite Assignment/Metabolic Profiling Analysis

Metabolite identification by MS was conducted by matching accurate m/z measurements of detected chromatographic peaks and molecular fragments.

Five experiments were performed for urine and serum analysis: NMR, reversed-phase UPLC-MS in ESI+ and ESI- modes, HILIC-UPLC-MS in ESI+ and ESI- modes. Three experiments were performed for each ulcer fluid sample: NMR, lipid profiling and reversed-phase UPLC-MS in ESI+ and ESI- modes.

### B - RESULTS

### Patient Demographics

Demographic and clinical details of patients that healed and failed to heal are presented in **Table 1.**

**Table 1: Patients demographics**

| | Healed (N=15) | Non-Healed (N=13) | *P* value |
|---|---|---|---|
| Age, years, (SD) | 73(12) | 71(16) | 0.7191 |
| Male, n (%) | 9(60) | 9(69) | 0.5024 |
| Ulcer Area, cm³, (SD) | 27(38) | 65(50) | 0.0333 |
| Ulcer Perimeter, cm, (SD) | 13.6(15.7) | 27.86(16.48) | 0.0281 |
| Ulcer Age, months, (SD) | 27(39) | 65(50) | 0.0333 |

**Table 1** shows that there is no statistically significant differences in age distribution or gender ratio between healed and non-healed groups analysed. However, there are significant differences in ulcer area, perimeter and age between healed and non-healed groups.

### Reversed Phase UPLC-MS analysis of serum, urine and ulcer fluid of the venous leg ulcer patients

Serum, urine and ulcer fluid samples were analysed using reversed phase UPLC-MS. The serum analysis revealed 8,296 metabolic features in the positive ionisation mode (ESI+), whilst 2,929 features were detected in the negative mode (ESI-). The urine examined showed 5,263 metabolic features in ESI+, whereas 7,026 features in ESI-. The ulcer fluid highlighted 16,457 metabolic features in ESI+ and 7,204 features in ESI-. Integrals of metabolic features with coefficient of variation (CV) more than 30% in the quality control (QC) samples were removed. Moreover, contaminant peaks in either QCs and blank controls were also excluded. The retained metabolic features were used for the final stages of analyses. Initial differences between healed and non-healed groups were highlighted when OPLS-DA models were performed.

The significance and robustness of the corresponding OPLS-DA of the serum (ESI-, p=0.0005 for R²Y=90% and Q²Y=57%), urine (ESI+, p=0.046 for R²Y=64% and Q²Y=25% and ESI-, p=0.024 for R²Y=87% and Q²Y=31%) and ulcer fluid (ESI-, p=0.047 for R²Y=58% and Q²Y=20%) were confirmed by cross-validated ANOVA (CV-ANOVA) testing; see **Table 2** and **Figure 1****.** The s-plot in SIMCA was used to visualise metabolic features that contributed to the differences in the significant OPLS-DA models. The integrals of the metabolic features responsible for the class discrimination (*i.e.* healed *vs* non-healed) were further subjected to statistical testing and the lipid profiling analysis of the serum has leaded the inventors to identify eight unique discriminant between the healed *versus* the non-healed group. Features included six ceramides (Cer d18:1/24:0, d18:1/24:1, d18:1/23:0, d18:2/23:0, d18:1/22:0, and d18:2/22:0), one ceramide-1-phosphate (CerP d18:1/18:0) and one sphingomyelin (SM d18:1/23:0). Overall, six different ceramide and ceramide-1-phosphate (CerP d18:1/18:0) intensities were significantly shown to be reduced in the healed patients versus the non-healed. SM d18:1/23:0 also showed significantly reduced intensities in the healed versus non healed group. Results are shown in **Table 2.**

**Table 2. Summary of model characteristics from significant OPLS-DA multivariate statistical analysis of all analysed data.**

| Sample type | Analyses | Components | R2X | R2Y | Q2 | CV ANOVA |
|---|---|---|---|---|---|---|
| Serum | HILIC-MS ESI+ | 1+2 | 47.20% | 86.00% | 43.90% | 6.66E-03 |
| | HILIC-MS ESI- | 1+2 | 24.70% | 96.60% | 34.20% | 3.76E-02 |
| | RPLC-MS ESI- | 1+1 | 35.50% | 90.40% | 57.10% | 5.25E-04 |
| Urine | RPLC-MS ESI+ | 1+1 | 24.20% | 64.40% | 24.90% | 4.55E-02 |
| | RPLC-MS ESI- | 1+2 | 27.30% | 87.40% | 31.90% | 2.78E-02 |
| Ulcer fluid | RPLC-MS ESI- | 1+1 | 30.30% | 58.10% | 19.70% | 4.72E-02 |

### HILIC-UPLC-MS analysis of serum, urine and ulcer fluid of the venous leg ulcer patients

Serum and urine samples were further examined using HILIC-UPLC-MS. Overall, 4,302 metabolic features were identified in the ESI+, whilst 3,623 features were observed in the ESI- of the run serum samples. The urine samples showed 15,637 metabolic features in the ESI+ and 5,491 metabolic features in ESI-. Similar to the RP-UPLC-MS analysis, metabolic peaks with coefficient of variance (CV)>30% across the QCs plus the contaminants' peaks in either the QCs or blanks were removed. This resulted in a remainder of 3,410 features for ESI+ and 2,442 features for the ESI- of the serum samples. In the urine samples, the ESI+ had 12,394 features and the ESI- had 3,701 features.

OPLS-DA modes were performed, and results are shown in **Figure 1****.** Differences were shown in the profiles derived from the serum samples when the corresponding OPLS-DA modes were performed. CV-ANOVA testing confirmed the robustness of the OPLS-DA models across the profiles generated from the serum (ESI+, p=0.006 for R²Y=86% and Q²Y=44% and ESI-, p=0.034 for R²Y=97% and Q²Y=34%). The SIMCA derived s-plot visualised the metabolic features highlighted by the significant OPLS-DA models generated from the serum HILIC-UPLC-MS profiles. Further statistical testing was performed on the integrals of the metabolic features that differentiate between classes (*i.e.* healed *vs* non-healed).

These analyses have shown that significant metabolic features included carnitine and phosphatidylethanolamine (PE 18:4/22:6). In total, the integral intensity of carnitine was significantly elevated in the healed group compared to the intensity of the non-healed. Furthermore, PE 18:4/22:6 intensity was significantly reduced in the healed patient serum samples compared to the non-healed samples.

### UPLC-MS metabolic profiling of serum

The metabolic profiles of serum were analysed using UPLC-MS. Results are shown in **Table 3.**

**Table 3. Discriminatory metabolites observed in serum via UPLC-MS**

| **Metabolite** | **Fold Change(H/NH)** | ***P* value** | **adjusted *P* value** |
|---|---|---|---|
| Carnitine | 1.840284485 | 8.76E-04 | 3.75E-02 |
| Cer(d18:1/24:0) | 0.790378672 | 3.18E-04 | 2.80E-02 |
| CerP(d18:1/18:0) | 0.782541073 | 9.10E-04 | 4.17E-02 |
| Cer(d18:2/23:0) | 0.781021223 | 3.35E-04 | 2.80E-02 |
| Cer(d18:1/22:0) | 0.744297674 | 2.45E-04 | 2.68E-02 |
| Cer(d18:1/24:1) | 0.738650889 | 1.23E-03 | 4.88E-02 |
| Cer(d18:1/23:0) | 0.722764682 | 1.97E-04 | 2.68E-02 |
| Cer(d18:2/22:0) | 0.656515972 | 2.32E-04 | 2.68E-02 |
| SM(d18:1/23:0) | 0.654286489 | 9.28E-04 | 4.17E-02 |
| PE (18:4/22:6) | 0.349720924 | 9.68E-04 | 3.75E-02 |

| | | | |
|---|---|---|---|
| Significance measured via Student's T-test with Benjamini-Hochberg multiple test correction Abbreviations: Cer, ceramide; CerP, ceramide-1-phosphate; SM, Sphingomyelin; PE, phosphatidylethanolamine; H, healers; NH, non-healers. | | | |

### Ratio of carnitine to ceramide as a serum biomarker predictive of ulcer healing

Serum-based carnitine to ceramide ratio has also been determined in patients. The results are shown in **Figure 2** and highlight that healed leg ulcer patients have a significantly (p-value <0.0001) elevated carnitine to ceramide ratio compared to non-healing patients.

### REFERENCES

Anwar, M.A. et al., 2015. Optimization of metabolite extraction of human vein tissue for ultra performance liquid chromatography-mass spectrometry and nuclear magnetic resonance-based untargeted metabolic profiling. Analyst, 140(22), pp.7586-7597.
Broszczak, D.A. et al., 2017. Molecular Aspects of Wound Healing and the Rise of Venous Leg Ulceration: Omics Approaches to Enhance Knowledge and Aid Diagnostic Discovery. The Clinical Biochemist Reviews, 38(1), p.35.
Callam, M.J. et al., 1985. Chronic ulceration of the leg: extent of the problem and provision of care. Br Med J (Clin Res Ed), 290(6485), pp.1855-1856.
Dona, A.C. et al., 2014. Precision high-throughput proton NMR spectroscopy of human urine, serum, and plasma for large-scale metabolic phenotyping. Analytical chemistry, 86(19), pp.9887-9894.
Ennis, W.J., Driscoll, D.M. & Meneses, P., 1994. A PRELIMINARY-STUDY ON P-31 NMR-SPECTROSCOPY-A POWERFUL TOOL FOR WOUND ANALYSIS USING HIGH-ENERGY PHOSPHATES. WOUNDS-A COMPENDIUM OF CLINICAL RESEARCH AND PRACTICE, 6(5), pp.166-173.
Gohel, M.S. et al., 2005. Randomized clinical trial of compression plus surgery versus compression alone in chronic venous ulceration (ESCHAR study)-haemodynamic and anatomical changes. British Journal of Surgery: Incorporating European Journal of Surgery and Swiss Surgery, 92(3), pp.291-297.
Gohel, M.S. et al., 2018. A randomized trial of early endovenous ablation in venous ulceration. New England Journal of Medicine*.*
Gottrup, F. et al., 2001. A new concept of a multidisciplinary wound healing center and a national expert function of wound healing. Archives of Surgery, 136(7), pp.765-772.
Hampton, S., 2016. The difficulty and the solution of compression therapy in a healed venous leg ulcer. British journal of community nursing, 21 (Sup9), pp.S34-S38.
Isaac, G., McDonald, S. & Astarita, G., 2011. Lipid separation using UPLC with charged surface hybrid technology. Waters App note*.*
James, T.J. et al., 2003. Evidence of oxidative stress in chronic venous ulcers. Wound Repair and Regeneration, 11(3), pp.172-176.
Junka, A. et al., 2017. Metabolic profiles of exudates from chronic leg ulcerations. Journal of pharmaceutical and biomedical analysis, 137, pp. 13-22.
Kalkhof, S. et al., 2014. Proteomics and metabolomics for in situ monitoring of wound healing. BioMed research international, 2014.
Mannello, F. et al., 2014. Omics profiles in chronic venous ulcer wound fluid: innovative applications for translational medicine. Expert review of molecular diagnostics, 14(6), pp.737-762.
Onida, S. & Davies, A.H., 2016. Predicted burden of venous disease. Phlebology, 31(1_suppl), pp.74-79.
Smith, C.A. et al., 2006. XCMS: processing mass spectrometry data for metabolite profiling using nonlinear peak alignment, matching, and identification. Analytical chemistry, 78(3), pp.779-787.
Spagou, K. et al., 2010. HILIC-UPLC-MS for exploratory urinary metabolic profiling in toxicological studies. Analytical chemistry, 83(1), pp.382-390.
Spencer et al., Stat Med. 2016 November 30; 35(27): 4909-4923.
Tautenhahn, R., Boettcher, C. & Neumann, S., 2008. Highly sensitive feature detection for high resolution LC/MS. BMC bioinformatics, 9(1), p.504.
Trengove, N.J., Langton, S.R. & Stacey, M.C., 1996. Biochemical analysis of wound fluid from nonhealing and healing chronic leg ulcers. Wound Repair and Regeneration, 4(2), pp.234-239.

## Claims

1. A method of diagnosis or prognosis of a non-healing or chronic wound comprising the step of determining the ratio of carnitine to ceramide and/or ceramide derivate in a sample from a mammalian, wherein said ratio is decreased when compared with a reference value.

2. A method of diagnosis or prognosis of a non-healing or chronic wound according to claim 1, comprising the following steps:
a) determining the ratio of carnitine to ceramide and/or ceramide derivate in a sample from a mammalian,
b) comparing the said ratio to a reference value,
c) providing a favorable diagnosis or prognosis of healing when the said ratio is higher than said reference value, or providing a poor diagnosis or prognosis of healing when the said ratio is lower than said reference value.

3. A method of diagnosis or prognosis of a non-healing or chronic wound according to any one of claims 1-2, wherein said reference value is 40.

4. A method of diagnosis or prognosis of a non-healing or chronic wound according to any one of claims 1-3, wherein said reference value has been determined in people who healed.

5. A method of diagnosis or prognosis of a non-healing or chronic wound according to any one of claims 1-4, wherein the said ceramide is Cer d18:1/24:0, Cer d18:1/24:1, Cer d18:1/23:0, Cer d18:2/23:0, Cer d18:1/22:0, and/or Cer d18:2/22:0.

6. A method of diagnosis or prognosis of a non-healing or chronic wound according to any one of claims 1-5, wherein the said ceramide derivative is a ceramide-1-phosphate, or a sphingomyelin.

7. A method of diagnosis or prognosis of a non-healing or chronic wound according to claim 6, wherein the said ceramide-1-phosphate is CerP d18:1/18:0.

8. A method of diagnosis or prognosis of a non-healing or chronic wound according to claim 6, wherein the said sphingomyelin is SM d18:1/23:0.

9. A method of diagnosis or prognosis of a non-healing or chronic wound according to any one of claims 1-8, wherein the said wound is an ulcer, preferably a leg ulcer, more preferably a venous leg ulcer or a chronic venous ulceration.

10. A method of diagnosis or prognosis of a non-healing or chronic wound according to any one of claims 1-9, wherein the said sample is chosen among: blood, serum, urine and ulcer fluids, preferably serum, urine and ulcer fluids, and more preferably serum.

11. A method of diagnosis or prognosis of a non-healing or chronic wound comprising the step of determining the level of at least one metabolite in a sample from a mammalian, wherein:
- at least carnitine is lower than a reference value, and/or
- at least one ceramide and/or one ceramide derivative is higher than a reference value, and/or
- at least one phosphatidylethanolamine is higher than a reference value.

12. A method of diagnosis or prognosis of a non-healing or chronic wound according to claim 11, wherein:
- the said ceramide is Cer d18:1/24:0, Cer d18:1/24:1, Cer d18:1/23:0, Cer d18:2/23:0, Cer d18:1/22:0, and/or Cer d18:2/22:0, and/or
- the said ceramide derivative is:
* a ceramide-1-phosphate, preferably CerP d18:1/18:0, and/or
* a sphingomyelin, preferably SM d18:1/23:0, and/or
- the said phosphatidylethanolamine is PE 18:4/22:6.
